(19) Europäisches Patentamt
European Patent Office
Office européen des brevets

(11) EP 2 546 652 A1

(12) EUROPEAN PATENT APPLICATION

(43) Date of publication:
16.01.2013 Bulletin 2013/03

(51) Int Cl.:
*G01N 33/543* (2006.01)     *G01N 33/569* (2006.01)

(21) Application number: 12172916.4

(22) Date of filing: 21.06.2012

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 14.07.2011 TW 100124898

(71) Applicant: MagQu Co. Ltd.
Taipei 231 (TW)

(72) Inventor: Yang, Shieh-Yueh
231 Taipei (TW)

(74) Representative: TER MEER - STEINMEISTER &
PARTNER GbR
Me/bk
Mauerkircherstrasse 45
81679 München (DE)

(54) **A testing kit for detecting aquatic animal virus and method thereof**

(57) The present invention provides a testing kit for detecting aquatic animal virus and a method thereof. The method uses a magnetic immunoassay analyzer to detect immunomagnetic reduction (IMR) signal of magnetic nanoparticles bounded with aquatic animal virus antibody. The method further determines the virus concentration in an aquatic animal through a logistic function, and ascertains if the aquatic animal to be infected.

Fig. 3

(a)

$Fe_3O_4$
Magnetic nucleus
Anti-NNV
FITC

(b)

(c)

# SEQUENCE LIST

<110> MagQu Co. LTD.

Yang, Shieh-Yueh

<120> A TESTING KIT FOR DETECTING AQUATIC ANIMAL VIRUS AND METHOD THEREOF

<130> 1754-MQ-EU

<160> 3

<170> PatentIn version 3.5

<210> 1

<211> 20

<212> DNA

<213> Artificial Sequence

<220>

<223> NNV specific primer

<400> 1

cgagtcaaca cgggtgaaga                                                                  20

<210> 2

<211> 19

<212> DNA

<213> Artificial Sequence


<220>

<223> NNV sense primer


<400> 2

gcccctgatg gagcagtct                                            19


<210> 3

<211> 22

<212> DNA

<213> Artificial Sequence


<220>

<223> NNV antisense primer


<400> 3

agcacggtca acatctccag tt


**Description**

**FIELD OF THE INVENTION**

The present invention relates to a testing kit for detecting aquatic animal virus and method thereof.

**DESCRIPTION OF PRIOR ART**

Grouper is an important type of fish which create high economy profit for countries in Asia and Europe; however, the breeding rate of larvae is very low due to larvae suffering from viruses (Kiryu et al., 2007, Fish Pathology 42, 163-165). There are several kinds of viruses which will attack groupers, such as nervous necrosis virus (NNV), irido virus and infection pancreatic necrosis virus (Munday et al., J. Fish Dis. 25, 127-142). Moreover, nervous necrosis virus (NNV) is the most harmful for larvae, and the mortality is higher than 90%.

Nervous necrosis virus (NNV) is one kind of nucleic acid virus which belongs to a type of beta-nodavirus. The size of NNV is about 25-30 nm, and NNV consists of two kinds of ribonucleic acid such as RAN 1 and RNA 2 (Tan et al., 2001, Singapore strain. J. Gen. Virol. 82, 647-653; Chi et al., 2001, J. Fish Dis. 24, 3-13; Sung et al., 2009, J. Virol. Methods 159, 206-210). In addition, RNA 1 can express protein A, while RNA 2 can express protein $\alpha$, forming the cap of nervous necrosis virus (Qin et al., 2002, Epinephelus spp. J. Virol. Method 106, 89-96; Adachi et al., 2008, Arch. Virol. 153, 15-24).

The common method for quantitatively detecting nervous necrosis virus comprises extracting NNV particles from grouper tissue, followed by reversing transcription from RNA to DAN, and finally using real-time PCR to detect nervous necrosis virus (Chi et al., 2003, Dis Aquat Org. 55, 221-228; Kuo et al., 2011, J. Clin. Microbiol. 49, 1090-1096). The above method can express high sensitivity and specificity during detecting NNV; however, the operation process is so delicate that the operator needs to be careful, and the method is only operated by the well-trained technicians at laboratory. The above disadvantage obstructs popularization of the method for detecting NNV seriously. In addition, in the field of aquaculture, the large-scale inspection of NNV is difficult to be achieved by real-time PCR. Currently, the most popular immunoassay technology is sandwich ELASA (Volpers et al., 1995, J. Virol. 6, 3258-3264; Breuil et al., 2001, Dis. Aquat. Org. 45, 25-31); however, the above immunoassay technology requires two kinds of antibodies against a ligand. The alternative technologies with merits of easy operation, high reliability, high sensitivity and specificity are currently developed due to the operation of the above method is complicated during inspection.

**BRIEF DESCRIPTION OF THE DRAWINGS**

Fig. 1 is a flow chart showing extraction of the nervous necrosis virus particles from grouper larvae.

Fig. 2 shows results of the virus concentration and extraction efficiency from the nervous necrosis virus through the extraction process of the present invention.

Fig. 3(a) shows a schematic view that the surface of the magnetic nanoparticle is bound with nervous necrosis virus antibody (Anti-NNV) and secondary antibody with fluorescein isothiocyanate (FITC) ; Fig. 3(b) and Fig. 3(c) show respectively movement status of nanoparticles by a magnet placed in the lower-right area, in which the fluorescent spot of FITC is observed by a fluorescent microscope.

Fig. 4 is a distribution diagram of the particle diameter of the magnetic nanoparticles bounded with anti nervous necrosis virus.

Fig. 5 shows that an imaginary part of ac magnetic susceptibility of the magnetic reagent acting as a function of the frequency of the applied field.

Fig. 6 shows a relationship between the NNV concentration $\phi_{NNV}$ and the IMR signals

Fig. 7 shows a curve diagram for the relationship between NNV concentration and the crossing point.

Fig. 8 shows the correlation between the immunomagnetic reduction (IMR) and the real-time PCR for detecting NNV concreation.

**SUMMARY OF THE INVENTION**

The present invention provides a testing kit for detecting aquatic animal virus, which comprises a solution; and a plurality of magnetic nanoparticles which are spread in the solution, wherein each magnetic nanoparticle comprises: a magnetic nucleus; a surfactant layer which covers the magnetic nucleus; and a plurality of aquatic animal virus antibodies which bind to the surfactant layer.

The present invention further provides a method for detecting aquatic animal virus, comprising steps of: (a) providing a testing kit of the present invention; (b) obtaining a sample of a brain tissue of an aquatic animal and extracting virus from the sample to provide an extract; (c) mixing the testing kit and the extract to provide a mixture, and measuring an immunomagnetic reduction signal by a magnetic immunoassay analyzer; and (d) quantifying a concentration of virus by a logistic function $$IMR(\%) = \frac{A-B}{1+(\frac{\phi_{NNV}}{\phi_o})^\gamma} + B \, ,$$ wherein A is a noise

intensity, B is a saturated parameter, $\phi_{NNV}$ is the concentration of the virus, $\phi_o$ is a parameter which is changed depending on $\phi_{NNV}$, and $\gamma$ is a fitting parameter.

## DETAILED DESCRIPTION OF THE INVENTION

The present invention provides a testing kit, which comprises a solution, wherein the solution is water; and a plurality of magnetic nanoparticles which are spread in the water, in which each magnetic nanoparticle comprises a magnetic nucleus; a surfactant layer which is coated in the magnetic nucleus; and a plurality of aquatic animal virus antibodies which is bound with the surfactant layer.

The term "surfactant" used herein, unless otherwise indicated, refers to a material that is able to significantly decrease the interfacial tension of a targeted solution, or decrease the interfacial tension between two solutions. A common surfactant is organic amphoteric substance with hydrophilic and hydrophobic group which could be soluble in an organic solution or water. In some embodiment of the present invention, the material of the surfactant layer is, but not be limited to, organic acid, protein A, protein G, dextran or liposome. In a preferred embodiment, the material of the surfactant is dextran.

In some embodiment of the present invention, the material of magnetic nanoparticle is selected from the group consisting of $Fe_3O_4$, $Fe_2O_3$, $MnFe_2O_4$, $CoFe_2O_4$ and $NiFe2O_4$. In a preferred embodiment, the material is $Fe_3O_4$.

In some embodiment of the present invention, the aquatic animal virus is nervous necrosis virus, irido virus or infection pancreatic necrosis virus. In the preferred embodiment, the aquatic animal virus is nervous necrosis virus.

In some embodiment of the present invention, the testing kit is used for detecting aquatic animal virus. In a preferred embodiment, the aquatic animal is grouper.

The present invention further provides a method for detecting aquatic animal virus, comprising steps of: (a) providing a testing kit of the present invention; (b) obtaining a sample of a brain tissue of an aquatic animal and extracting virus from the sample to provide an extract; (c) mixing the testing kit and the extract to provide a mixture, and measuring an immunomagnetic reduction signal by a magnetic immunoassay analyzer; and (d) quantifying a concentration of virus by a logistic function $$IMR(\%) = \frac{A-B}{1+(\frac{\phi_{NNV}}{\phi_o})^\gamma} + B \, ,$$ wherein A is a noise intensity, B is a saturated parameter, $\phi_{NNV}$

is the concentration of the virus, $\phi_o$ is a parameter which is changed depending on $\phi_{NNV}$, and $\gamma$ is a fitting parameter.

In some embodiment of the present invention, the present invention further provides the method of extracting virus of step(b) comprises the steps of: (a) putting the brain tissue in a container and placing the container on ice; (b) adding an extraction solution to the tissue in the container; (c) grinding the tissue in the container; and (d) collecting an supernatant from the container, and detecting an efficiency of an extraction. In the preferred embodiment of the present invention, the extraction is virus.

In some embodiment of the present invention, the virus is nervous necrosis virus, irido virus or infection pancreatic necrosis virus. In a preferred embodiment, the virus is nervous necrosis virus.

In some embodiment of the present invention, a range of efficiency of the extraction is selected from 700 $TCID_{50}$/ml to $4\times10^7$ $TCID_{50}$/ml.

In some embodiment of the present invention, a formula of calculating the immunomagnetic reduction (IMR) signal is IMR (%) = ($\chi_{ac,o}$ - $\chi_{ac,\phi}$)/$\chi_{ac,o}$ X 100 %.

Unless defined otherwise, all technical and scientific terms used herein have the same meaning as commonly understood to one of ordinary skill in the art to which this invention pertains. In this application, certain terms are used, which shall have the meanings as set in the specification. It must be noted that as used herein and in the appended claims, the singular forms "a," "an," and "the" include plural reference unless the context clearly dictates otherwise.

## EXAMPLES

The examples below are non-limiting and are merely representative of various aspects and features of the present invention.

**EXAMPLE 1: EXTRACTING NERVOUS NECROSIS VIRUS FROM GROUPER LARVAE**

The nervous necrosis virus (NNV) almost exists in the brain of grouper. Thus, in the present embodiment, it was first to take out a sample of 0.5g brain tissue from the grouper and put the sample into an enppendorf, and added 200$\mu$l extraction buffer (MagQu Co., Ltd.) into the enppendorf. In order to keep the sample in freshness, the enppendorf was put on a piece of ice and the sample was grinded for 3 minutes. Then, the enppendorf was removed from the ice for 5 minutes. A supernatant was collected from the enppendorf for measuring an immunomagnetic reduction signal by a magnetic immunoassay analyzer to obtain a concentration of the nervous necrosis virus.

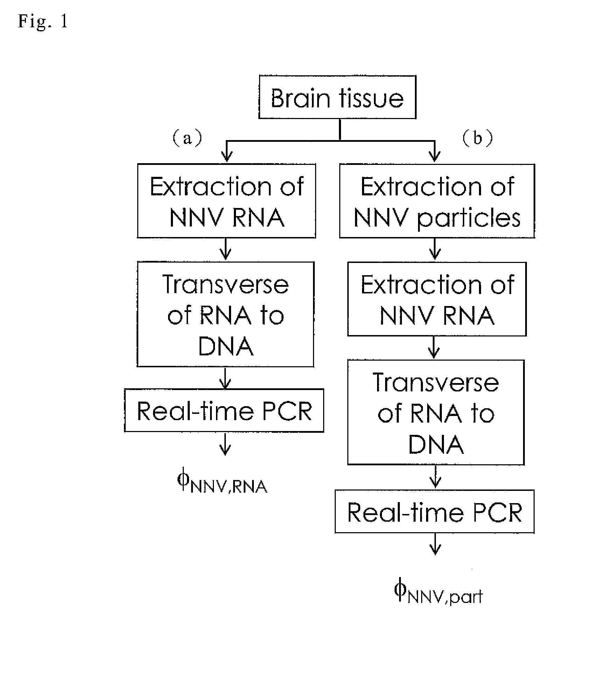

Referring to Fig. 1 that showed a flow chart of an extraction process from brain tissues of different groupers. The process of Fig. 1(a) showed that RNA of the nervous necrosis virus was extracted directly from the brain tissue of the larvae, and the extracted RNA was transcribed into DNA. Finally, the DNA concentration was detected by a real-time polymerase chain reaction. In the present invention, the NNV concentration was denoted $\phi_{NNV, PCR}$. Based on results from the extraction, a range of an NNV concentration was selected from 700 $TCID_{50}$/ml to $4x10^7$ $TCID_{50}$/ml for surveying the extracted NNV concentration efficiency from larvae. The term "$TCID_{50}$" used herein refers to tissue culture inflection $dose_{50}$.

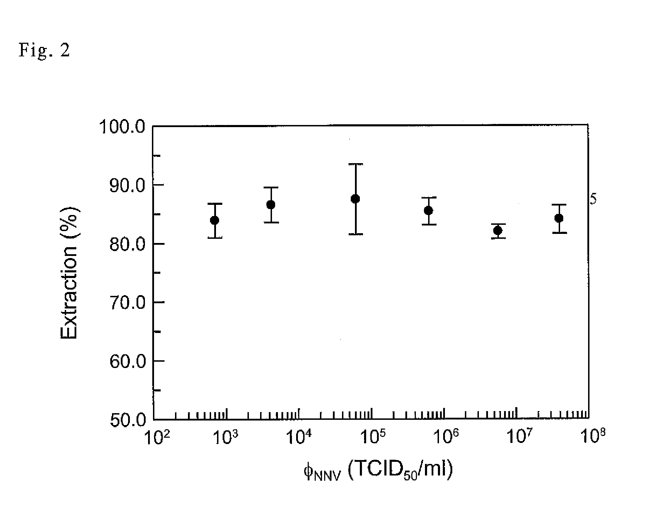

On the other hand, the process of Fig. 1(b) showed that the NNV particles in the brain tissue were extracted. Through the above extraction method, the supernatant containing NNV particles was detected by the real-time polymerase chain reaction which was denoted as $\phi_{NNV}$, part- Furthermore, the NNV extraction efficiency could be obtained from the ration of $\phi_{NNV, part}$ / $\phi_{NNV, PCR}$. As shown in Fig. 2, the NNV extraction efficiency from larvae is more than 80% for the NNV concentration from 700 $TCID_{50}$/ml to $4x10^7$ $TCID_{50}$/ml.

**EXAMPLE 2: SYNTHESIS OF TESTING KIT (REAGENT)**

A ferrite solution containing ferrous sulfate hepta-hydrate ($FeSO_4 \cdot 7H_2O$) and ferric chloride hexa-hydrate ($FeCl_3 \cdot 6H_2O$) with a stoichiometric ratio of 1:2. The ferrite solution was mixed with an equal volume of aqueous dextran which was acted as surfactant for $Fe_3SO_4$ particles to be dispersed in water. The above mixer was heated to 70°C~90°C and titrated with strong base solution to form black $Fe_3SO_4$ particles. Aggregations and excess unbound dextran were removed by centrifugation to obtain high concentration homogeneous magnetic fluid through a gel filtration chromatography. The testing kit could obtain a desired magnetic concentration by diluting the high concentration magnetic fluid with pH7.4 phosphate buffered saline (PBS) solution (Jiang et al., 2004, J. Magn. Magn. Mater. 283, 210-214).

In order to bind antibodies (i.e. anti-nervous necrosis virus) on an outer shell of the dextran of magnetic nanoparticles, taking anti-nervous necrosis virus as an sample, a $NaIO_4$ solution was added into the magnetic solution to oxidize dextran and then create aldehyde group (-CHO) (Yang et al., 2008, Magn. Magn. Mater. 320, 2688-2691). Then, the dextran could have reaction with the anti-nervous necrosis virus by the linking of -CH=N-, therefore, anti-nervous necrosis virus was bound to dextran by covalent bond. Through magnetic separation, unbound anti-nervous necrosis virus could be separated from the solution.

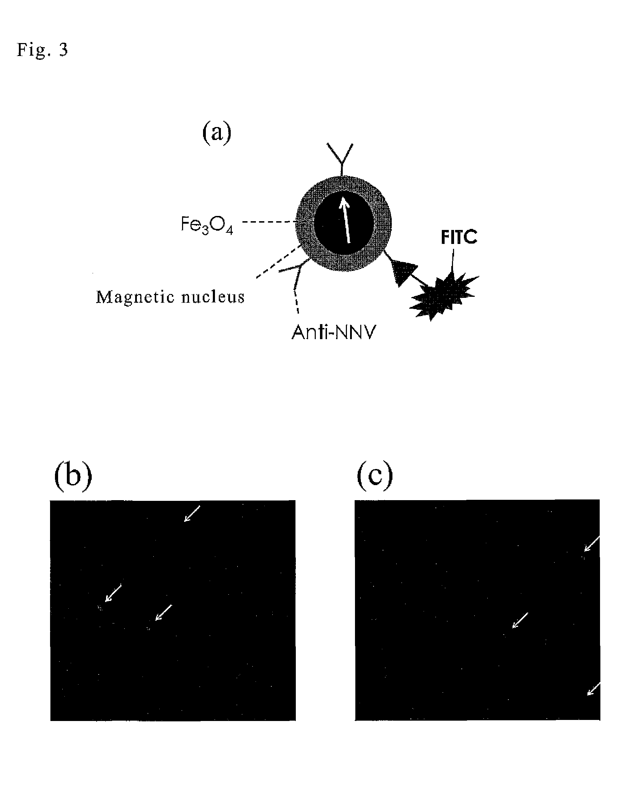

In order to confirm that the surface of the magnetic nanoparticles was bound with nervous necrosis virus antibodies. As shown in Fig. 3(a) which showed that secondary antibodies with fluorescein isothiocyanate (FITC) were mixed with the NNV reagent. In the experiment, the excess secondary antibodies with FITC were mixed with the magnetic nanoparticles which coated nervous necrosis virus antibodies. After mixing fully, the magnetic nanoparticles were removed from the reagent through the magnetic sepatation technology; on the other hand, the other secondary antibodies with FITC that did not bind with the surface of magnetic nanoparticles with NNV antibodies, the secondary antibodies with FITC could remain in the reagent due to not providing with magnetism.

In this embodiment, a droplet of the reagent was placed on a glass plate, and the fluorescent spots of FITC were observed by a fluorescent microscope. As shown in Fig. 3(b), the green spots were labeled with arrows. The light was produced from FITC. In order to confirm that the secondary antibodies were bound with the magnetic nanoparticles once again, a magnet was placed beside the droplet reagent to attract the magnetic nanoparticles in the reagent. Through movement of the magnetic nanoparticles, the spots will move if the secondary antibodies with FITC and the magnetic nanoparticles which were coated with NNV antibodies bound. In this embodiment, the magnet was placed at a right side on the bottom of the droplet reagent as shown in Fig. 3(b), these green spots should move toward to the bottom right by magnet as shown in Fig. 3(c). As mentioned above, the magnetic nanoparticles were definitely bound with anti-nervous necrosis virus.

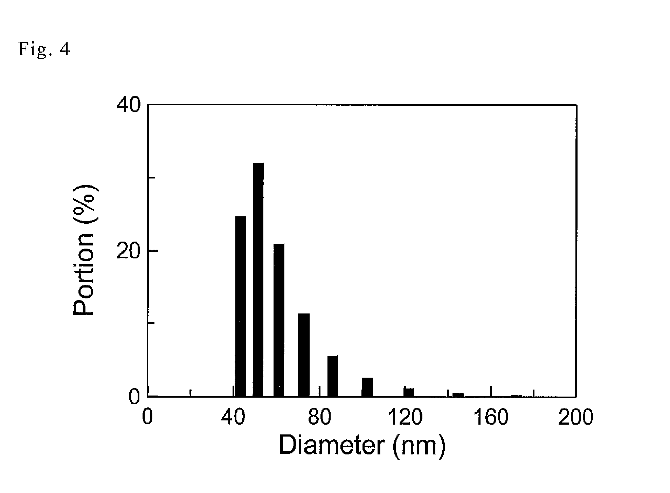

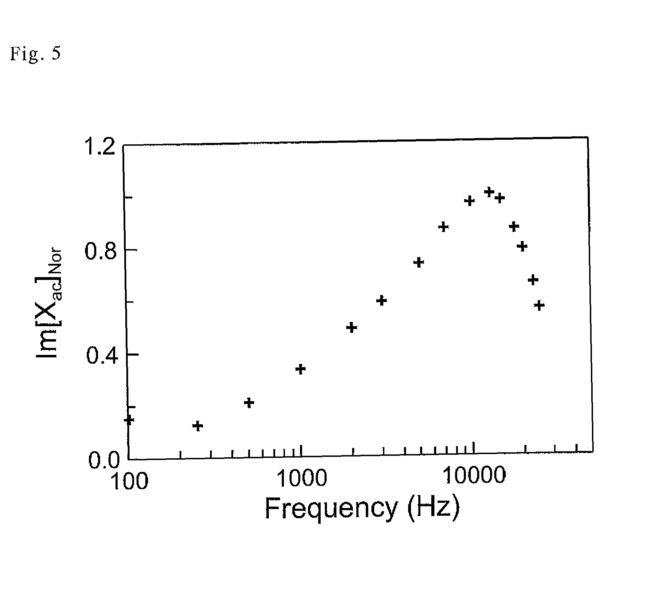

In this embodiment, a diameter distribution of the magnetic nanoparticles with anti-nervous necrosis virus was analyzed by a dynamic laser scattering. The magnetic concentration of the testing kit (reagent) for nervous necrosis virus was measured to be 0.1 emu/g (1.2 mg-Fe/ml). As shown in Fig. 4, a mean hydrodynamic diameter of particles was about 55 nm. According to Brownian relaxation, the time constant $\tau_B$ for the particles diameter was 55 nm and being dispersed in water at 20-25 °C was estimated to be 65.79 $\mu$s via $\tau_B = 3\eta V/k_B T$ (formula 1), wherein $\eta$ is a viscosity of water at 20-25 °C, V is the mean hydrodynamic volume of particles, $k_B$ is Boltzmann constant, and T is a Kelvin temperature. Through estimation above, this implied that a frequency (resonant frequency) for oscillation of particles under an ac (alternating current) magnetic field was $1/\tau_B$ =15.2 kHz. Physically, an imaginary part of the ac magnetic susceptibility of the particles dispersed in water corresponded to absorption of the applied ac magnetic energy of the particles. As mentioned above, for a resonant frequency, it could reach maximum absorption, and the imaginary part of ac magnetic susceptibility reached the maximum value. In the embodiment, the frequency versus the imaginary part of the ac magnetic susceptibility of the NNV testing kit (reagent) was measured by a magnetosusceptometer. Fig. 5 showed a normalized maximum value of the imaginary part of the ac magnetic susceptibility within an experimental frequency range, and showed obviously that the imaginary part $Im[\chi_{ac}]_{Nor}$ of the NNV testing kit (reagent) increased with increasing the frequency of the ac magnetic field, and the maximum value reached about 15kHz. The above experimental result was consistent with the deduction from formula 1 ($\tau_B = 3\eta V/k_B T$). In the following IMR measurement, in order to optimize the magnetic response of NNV testing kit (reagent), the frequency of the applied ac field was about 15kHz.

**EXAMPLE 3: BUILDING THE RELATIONSHIP FROM CONCENTRATION OF ANTI-NERVOUS NECROSIS VIRUS AND IMMUNOMAGNETIC REDUCTION (IMR) SIGNAL**

In this embodiment, a 40μl testing kit (reagent) and a 60μl sample solution were mixed in a glass tube, while measuring two kinds of $\chi_{ac}$ signals by a magnetic immunoassay analyzer (XacPro-E, MagQu) which comprises $\chi_{ac,0}$ and $\chi_{ac,\phi}$, wherein the $\chi_{ac,0}$ represented the ac signal for a mixture not being formed an immuno complex of NNV-anti-NNV-dextran-magnetic particle and $\chi_{ac,\phi}$ represented the ac signal for the mixture being formed the NNV-anti-NNV-dextran-magnetic particle at a room temperature. In this embodiment, using the following formula 2 to calculate IMR signal by recordings of $\chi_{ac,0}$ and $\chi_{ac,\phi}$.
$$IMR\ (\%) = (\chi_{ac,0} - \chi_{ac,\phi})/\chi_{ac,0} \times 100\ \% \cdots Formula\ 2$$

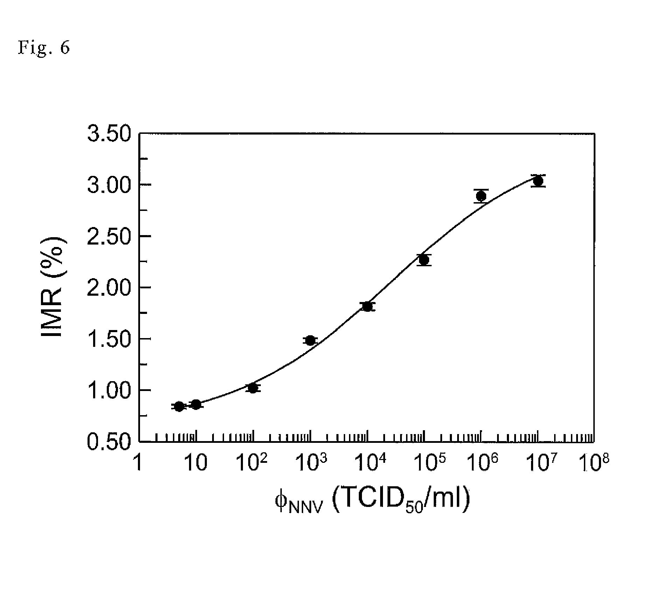

In this embodiment, for a sample of a given concentration, the time-dependence $\chi_{ac}$ signal was detected triply. Referring to Fig. 6 that showed a relationship between the NNV concentration $\phi_{NNV}$ and the IMR signals. The IMR signal increased from 0.84% to 3.04% while the NNV concentration $\phi_{NNV}$ changed from 5 TCID$_{50}$/ml to 10$^7$ TCID$_{50}$/ml.
Based on the experimental results above, the relationship between the IMR (%) and NNV concentration $\phi_{NNV}$ was calculated by the following logistic function (formula 3), wherein A, B, $\phi_0$ and $\gamma$ were fitting parameters. The experimental data was introduced into the formula 3, thereby obtaining A=0.64, B=3.47, $\phi_0$ =26684 and $\gamma$ =0.31, which were drawn in Fig. 6 by a solid line. In the logistic function (formula 3), the IMR signal approximated to A value as $\phi_{NNV}$ approached zero. The result showed that A was a noise intensity that the NNV concentration was dependent on the IMR signal. In addition, as $\phi_{NNV}$ was higher than $\phi_0$, the IMR signal approximated to B value. This result showed that as NNV had a higher concentration, B value was a high-end value for IMR signal.
$$IMR(\%) = \frac{A-B}{1+(\frac{\phi_{NNV}}{\phi_o})^\gamma} + B \cdots Formula\ 3$$

As mentioned above, the concentration was defined as a standard of the detected sample. For NNV having a low concentration, through data of three repetitive standard deviations, it showed that the IMR signal was higher than A value of the noise level. Moreover, the experimental result showed that the standard deviation of IMR signal was 0.021 % for the low NNV concentration. Thus, in light of formula 3, when the standard deviation of the IMR signal was 0.903%, the NNV concentration was 17.2 TCID$_{50}$/ml.

**EXAMPLE 4: DETECTING THE CONCENTRATION OF NERVOUS NECROSIS VIRUS BY REAL-TIME PCR**

In this embodiment, after taking out 100ng RNA of nervous necrosis virus, and using an ABI Prism 7000 sequence detector (Applied Biosystem) to detect the sample. In addition, performing an amplification action of real-time PCR used a high capacity cDNA Archive kit (Applied Biosystem) and specific primer, such as SEQ ID NO.1. In RT-PCR process, the nucleic acids were detected by ABI real-time PCR containing Power SYBR® Green PCR MasterMix, and using a sense primer and antisense primer of nervous necrosis virus, such as SEQ ID NO.2 and SEQ ID NO.3 to perform real-time PCR. The amplification parameters included 45 cycles of reaction with 30 sec denaturing at 95°C, 30 sec annealing and extension at 60°C. Finally, the signal of real-time PCR was analyzed by SDS software (version 1.7; Applied Biosystem).

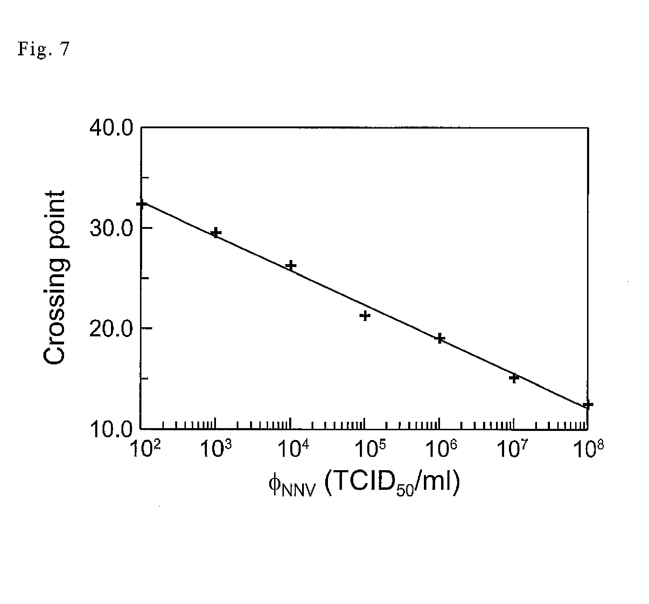

In this embodiment, samples of various NNV concentrations from $10^2$ to $10^8$ $TCID_{50}$/ml were prepared, while using the real-time PCR to detect. A crossing point (CP) for each NNV concentration sample was determined by experimental correlation between fluorescent intensity and the amplifying cycle. Fig.7 showed that a curve diagram for the relationship between NNV concentration and the crossing point. Moreover, a linear formula of the crossing point was obtained, such as the following formula 4, which acted as a standard curve for assaying NNV concentrations.

$$\text{Crossing point (CP)}= -1.48 \log \phi_{NNV} + 39.4 \cdots \text{ Formula 4}$$

**EXAMPLE 5: COMPARING ANALYSIS METHODS WITH IMMUNOMAGNETIC REDUCTION (IMR) SIGNAL AND RT-PCR**

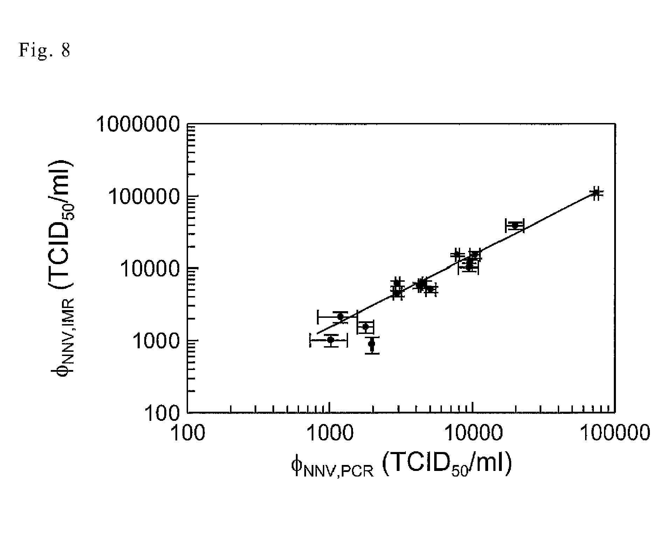

In order to confirm that the IMR analysis method for detecting NNV concentration of the present invention was able to substitute for the real-time PCR analysis method, in this embodiment, 15 samples for NNV assay were detected by the IMR and the real-time PCR simultaneously. For each sample, there were two values of NNV concentrations, one value being detected by IMR, which denoted as $\phi_{NNV,IMR}$, the other value was detected by using real-time PCR, which denoted as $\phi_{NNV,PCR}$. Fig.8 showed a high correlation between $\phi_{NNV,IMR}$ and $\phi_{NNV,IMR}$ was 0.99. As mentioned above, it is verified that using immunomagnetic reduction (IMR) assay method for quantitatively detecting NNV concentration was reliable.

All publications and patent applications cited in the present specification are herein incorporated by reference as if each individual publication or patent application were specifically and individually indicated to be incorporated by reference.

The present invention has been described in terms of particular embodiments found or proposed by the present inventor to comprise preferred modes for the practice of the invention. It will be appreciated by those of skill in the art that, in light of the present disclosure, numerous modifications and changes can be made in the particular embodiments exemplified without departing from the intended scope of the invention. Moreover, due to biological functional equivalency considerations, changes can be made in protein structure without affecting the biological action in kind or amount. All such modifications are intended to be included within the scope of the appended claims.

**Claims**

1. A testing kit for detecting aquatic animal virus, comprising:

  a solution; and
  a plurality of magnetic nanoparticles which are spread in the solution, and each magnetic nanoparticle comprises:

a magnetic nucleus;
a surfactant layer which covers the magnetic nucleus; and
a plurality of aquatic animal virus antibodies which bind to the surfactant layer.

2. The testing kit of claim 1, wherein the solution comprises water.

3. The testing kit of claim 1, wherein the material of magnetic nanoparticle is selected from the group consisting of $Fe_3O_4$, $Fe_2O_3$, $MnFe_2O_4$, $CoFe_2O_4$ and $NiFe_2O_4$.

4. The testing kit of claim 1, wherein the material of magnetic nanoparticle is $Fe_3O_4$.

5. The testing kit of claim 1, wherein the surfactant layer is organic acid, protein A, protein G, dextran or liposome.

6. The testing kit of claim 1, wherein the surfactant layer is dextran.

7. The testing kit of claim 1, wherein the aquatic animal virus is nervous necrosis virus, irido virus or infection pancreatic necrosis virus.

8. A method for detecting aquatic animal virus, comprising steps of:

   (a) providing a testing kit as claimed in claim 1;
   (b) obtaining a sample of brain tissue of an aquatic animal and extracting virus from the sample to provide an extract;
   (c) mixing the testing kit and the extract to provide a mixture, and measuring an immunomagnetic reduction (IMR) signal of the mixture by a magnetic immunoassay analyzer; and

   (d) quantifying a concentration of virus by a logistic function $$IMR(\%) = \frac{A - B}{1 + (\frac{\phi_{NNV}}{\phi_o})^\gamma} + B,$$ wherein A is a noise intensity, B is a saturated parameter, $\phi_{NNV}$ is the concentration of the virus, $\phi_o$ is a parameter which is changed depending on $\phi_{NNV}$, and $\gamma$ is a fitting parameter.

9. The method of claim 9, wherein the method of extracting virus of step(b) comprises the steps of:

   (a) putting the brain tissue in a container and placing the container on ice;
   (b) adding an extraction solution to the tissue in the container;
   (c) grinding the tissue in the container; and
   (d) collecting an supernatant from the container, and detecting an efficiency of the extraction.

10. The method of claim 9, wherein the aquatic animal virus is nervous necrosis virus, irido virus or infection pancreatic necrosis virus.

11. The method of claim 9, wherein the aquatic animal virus is nervous necrosis virus.

12. The method of claim 9, wherein a range of efficiency of the extraction is selected from 700 $TCID_{50}$/ml to $4 \times 10^7$ $TCID_{50}$/ml.

13. The method of claim 9, wherein a formula of calculating the immunomagnetic reduction (IMR) signal is IMR (%) = $(\chi_{ac,o} - \chi_{ac,\phi})/\chi_{ac,o} \times 100$ %.

Fig. 1

Fig. 2

Fig. 3

(a)

Fe$_3$O$_4$ ----

Magnetic nucleus

Anti-NNV

FITC

(b)

(c)

Fig. 4

Fig. 5

Fig. 6

Fig. 7

Fig. 8

Europäisches
Patentamt

European
Patent Office

Office européen
des brevets

# EUROPEAN SEARCH REPORT

Application Number

EP 12 17 2916

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WANG C H ET AL: "An integrated microfluidic loop-mediated-isothermal-amplification system for rapid sample pre-treatment and detection of viruses", BIOSENSORS AND BIOELECTRONICS, ELSEVIER BV, NL, vol. 26, no. 5, 15 January 2011 (2011-01-15), pages 2045-2052, XP027580115, ISSN: 0956-5663 [retrieved on 2010-09-09] * the whole document * * In particular: Title; Abstract; Materials and methods section; Fig. 1. * ----- | 1-13 | INV. G01N33/543 G01N33/569 |
| Y | US 3 970 518 A (GIAEVER IVAR) 20 July 1976 (1976-07-20) * the whole document * ----- | 1-13 | |
| Y | "Sea bass Dicentrarchus labrax nervous necrosis virus isolates with distinct pathogenicity to sea bass larvae", , 1 January 2001 (2001-01-01), XP55037001, Retrieved from the Internet: URL:http://www.int-res.com/articles/dao/45/d045p025.pdf [retrieved on 2012-09-03] * the whole document * * In particular: Title; Abstract; Materials and methods section. * ----- | 1-13 | TECHNICAL FIELDS SEARCHED (IPC) G01N |

The present search report has been drawn up for all claims

| Place of search | Date of completion of the search | Examiner |
|---|---|---|
| The Hague | 3 September 2012 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**EP 2 546 652 A1**

## ANNEX TO THE EUROPEAN SEARCH REPORT
## ON EUROPEAN PATENT APPLICATION NO.

EP 12 17 2916

This annex lists the patent family members relating to the patent documents cited in the above-mentioned European search report.
The members are as contained in the European Patent Office EDP file on
The European Patent Office is in no way liable for these particulars which are merely given for the purpose of information.

03-09-2012

| Patent document cited in search report | Publication date | Patent family member(s) | Publication date |
|---|---|---|---|
| US 3970518 A | 20-07-1976 | NONE | |

For more details about this annex : see Official Journal of the European Patent Office, No. 12/82

EPO FORM P0459

19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KIRYU et al.** *Fish Pathology,* 2007, vol. 42, 163-165
- **MUNDAY et al.** *J. Fish Dis.,* vol. 25, 127-142
- **TAN et al.** *Singapore strain. J. Gen. Virol.,* 2001, vol. 82, 647-653
- **CHI et al.** *J. Fish Dis.,* 2001, vol. 24, 3-13
- **SUNG et al.** *J. Virol. Methods,* 2009, vol. 159, 206-210
- **QIN et al.** *Epinephelus spp. J. Virol. Method,* 2002, vol. 106, 89-96
- **ADACHI et al.** *Arch. Virol.,* 2008, vol. 153, 15-24
- **CHI et al.** *Dis Aquat Org.,* 2003, vol. 55, 221-228
- **KUO et al.** *J. Clin. Microbiol.,* 2011, vol. 49, 1090-1096
- **VOLPERS et al.** *J. Virol.,* 1995, vol. 6, 3258-3264
- **BREUIL et al.** *Dis. Aquat. Org.,* 2001, vol. 45, 25-31
- **JIANG et al.** *J. Magn. Magn. Mater.,* 2004, vol. 283, 210-214
- **YANG et al.** *Magn. Magn. Mater.,* 2008, vol. 320, 2688-2691